# EUROPEAN PATENT APPLICATION

(11) **EP 4 656 237 A1**
(43) Date of publication of application: **03.12.2025**
(21) Application number: 25170968.9
(22) Date of filing: 16.04.2025
(51) Int. Cl.: A61N 5/10

(54) **SPOT DELIVERY VISUALIZATION FOR PARTICLE BEAM RADIOTHERAPY**

(30) Priority: 31.05.2024 US 202418680482
(71) Applicant: Elekta Inc., Atlanta, GA 30346 (US)
(72) Inventor: GENET, Louis Arunus, Atlanta, 30346 (US)
(74) Representative: Haseltine Lake Kempner LLP

(57) **Abstract**

Systems and methods for presenting visual results of a particle beam treatment delivery, including visualizing a deviation for an amount and a location of a particle beam treatment dose, are discussed. An example system may: obtain planning information of a planned particle beam treatment, as planned to be provided to treatment spots located in a treatment area of a patient; obtain delivery information of an actual delivered particle beam treatment to the treatment spots; and determine an applicable dose deviation between a planned amount of radiation and a delivered amount of radiation for each of the treatment spots. The system may present a visualization that represents the treatment spots and the applicable dose deviation, at each of the treatment spots, such as with a three-dimensional representation that uses colors or shading to identify the applicable dose deviation.

## Description

### TECHNICAL FIELD

Embodiments herein relate to methods and systems for processing medical data. In particular, the methods and systems are directed to processing medical data associated with radiotherapy.

### BACKGROUND

Radiotherapy or radiation therapy can be described as the use of ionizing radiation to damage or destroy unhealthy cells in both humans and animals. Unhealthy cells may include cancerous cells, for example. Radiation may be referred to as "prescribed" because generally a physician orders a predefined dose of radiation to be delivered to a targeted region such as a tumor on the surface of the skin or deep inside the body.

Common forms of ionizing radiation include X-rays and charged particles. One example of a radiotherapy technique is referred to as a "gamma knife" where a patient is irradiated using a number of lower-intensity gamma rays that converge with higher intensity and high precision at a targeted region. Another example of radiotherapy comprises using a linear accelerator ("linac"), whereby a targeted region is irradiated by high-energy particles (e.g., electrons, high-energy photons, and the like).

In another example, radiotherapy is provided using a heavy charged particle accelerator (e.g., protons, carbon ions, and the like), which is commonly referred to as "particle therapy" or "particle beam therapy". One significant known advantage of proton therapy is that it provides superior dose distribution with minimal exit dose compared to other forms of radiation therapy, such as x-ray therapy. This provides a significant reduction of dose to organs at risk (OAR) and other healthy tissue because of the minimal exit dose. Further advantages of particle beam therapy include lower dose per treatment, which lowers the risk of side effects and may improve quality of life during and after treatment.

The industry standard for particle therapy delivery is Pencil Beam Scanning (PBS). PBS uses magnets to steer the proton beam, delivering numerous 'spots' of radiation that make up a customized, three-dimensional delivery shape. This dose shape can conform to the specific shape of a tumor to destroy cancer cells while preserving healthy tissue nearby. Thus, a radiotherapy treatment controls the placement and dose of the radiation beam to provide a prescribed dose of radiation to a targeted region, while attempting to reduce or minimize damage to the OARs and the surrounding healthy tissue.

### BRIEF DESCRIPTION OF DRAWINGS

The patent or application file contains at least one drawing executed in color. Copies of this patent or patent application publication with color drawing(s) will be provided by the Office upon request and payment of the necessary fee.

Systems and methods in accordance with non-limiting examples will now be described with reference to the accompanying figures in which:
**FIG. 1** depicts a system adapted for implementing particle therapy and related aspects using the approaches discussed herein.
**FIG. 2** depicts a radiation therapy system operable in connection with a particle treatment system and an imaging acquisition device, according to an example.
**FIG. 3** depicts an example operational flow of a particle treatment system, according to an example.
**FIGS. 4** **and** **5** depict radiation dose depths and dose curves for various types of particles and treatments, according to an example.
**FIG. 6** depicts Pencil Beam Scanning of an irregular shape volume, according to an example.
**FIG. 7** depicts a diagrammatic representation of an example active scanning proton beam delivery system, according to an example.
**FIG. 8A** **and** **FIG. 8B** depict treatment spots and dose deviation provided by a particle therapy, according to an example.
**FIG. 9A****,** **FIG. 9B****, and** **FIG. 9C** depict another representation of treatment spots and dose deviation provided by a particle therapy, according to an example.
**FIG. 10A** **and** **FIG. 10B** depict treatment spots and dose location deviation provided by a particle therapy, according to an example.
**FIG. 11A****,** **FIG. 11B****, and** **FIG. 11C** depict another representation of treatment spots and dose location deviation provided by a particle therapy, according to an example.
**FIG. 12** depicts a flowchart of an example method for visualizing results of a particle beam treatment delivery, based on the approaches discussed herein.
**FIG. 13** is a block diagram of an apparatus, device, system, or machine for implementing computerized aspects of the various embodiments discussed herein.

### DETAILED DESCRIPTION

The following describes methods and systems that improve the operation and verification of particle therapy systems and treatments, based on a visualization system configured to illustrate differences between planned and delivered radiation doses. A visualization system can be adapted to show deviation from a planned particle beam treatment based on deviation in amount, location, or other characteristics, relative to respective areas or "spots" of treatment of a patient anatomy that were exposed to the particle beam therapy. Among other benefits, this visualization enables users to audit treatments for low quality delivery, which can inform dosage adjustments for future fractions. Additionally, early identification of therapy delivery inaccuracy may prompt a site's medical physicists to fine tune calibration of the delivery device, or identify other changes for the particle beam therapy.

As an overview of treatment planning, computer systems are used to create treatment plans that control and personalize the output of particle beam therapy to a particular patient. The software applications that create and modify treatment plans are referred to as a "treatment planning system" (or TPS), and these systems often include sophisticated functionality to calculate, customize, verify, and optimize the details of a specific radiotherapy treatment and the treatment plan data. For particle therapy, the TPS is used to calculate a specific three-dimensional map of spots required to treat a patient's tumor. Some TPS implementations include the capability to visualize the individual location of spots within the 3D space of the patient's CT scan during treatment planning (e.g., before the treatment is actually delivered). Each spot has a specific dosage of radiation calculated and planned by the TPS to effectively treat the tumor at that spot location.

Once the patient's plan is complete and approved, it is commonly sent to a software system known as Oncology Informatics System (OIS). The OIS stores the plan and communicates it to the radiotherapy delivery device (e.g., the particle therapy machine). The radiotherapy delivery device then delivers each spot as accurately as it can, returning a treatment record to the OIS containing information on the dose delivered at each spot, as well as the accuracy of the spot's position. Due to a variety of factors involved in particle therapy, some deviation may occur between the originally planned spot and the actually delivered spot.

Although some OIS implementations include the functionality to identify if there is a discrepancy between planned dose versus delivered dose in a treatment field, this is performed at a level of tracking an entire treatment field, and not at a level of tracking individual spots of delivery. Accordingly, there is a need for improved methods and systems for tracking and understanding such deviations for particle beam treatments at individual spots or locations of delivery, to improve the accuracy of therapy, to more accurately represent the outcomes of therapy, and to improve the computation operations that result in implementing systems.

The following methods and systems thus introduce visualization functionality, e.g., within the OIS or a related information system, to provide a 3D representation of the delta (difference) between the planned arrangement of treatment spots versus the delivered arrangement of treatment spots. This visualization may be provided as a three-dimensional visual array that is generated by comparing spot information from the treatment plan and the treatment record from the delivery device. The discrepancy or delta in expected versus delivered treatment levels may relate to deviation of the amount of dose, deviation of the direction or location of the dose, or other measurable characteristics. This visualization enables spot-level analysis of a treatment's accuracy, and as noted can help identify incorrectly calibrated equipment, operational errors, and related conditions.

Further explanation of this particle beam treatment and spot delivery visualization functionality is provided after an overview of radiotherapy particle beam treatment and treatment planning systems generally.

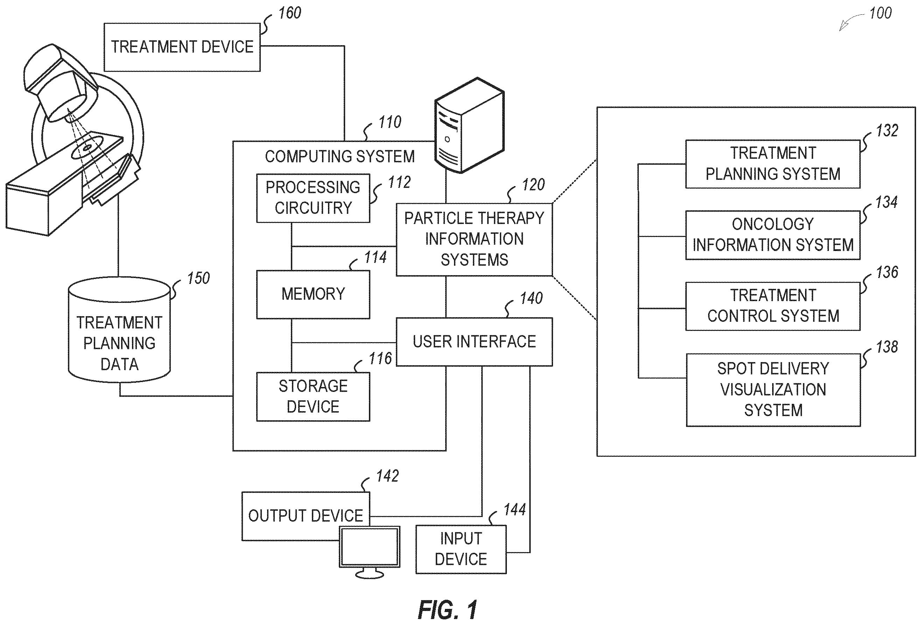

**FIG. 1** illustrates generally an example of a radiotherapy system 100, such as may be used for implementing particle therapy and related aspects of the present disclosure, in accordance with an example. This radiotherapy system 100 and its subsystems are described at a high level, and provide only a brief summary of the variations and use cases of treatment planning and treatment, involving many data optimization, visualization, and other medical evaluative and diagnostic operations.

The radiotherapy system 100 includes a radiotherapy data processing computing system 110 that hosts multiple particle therapy information systems 120. The radiotherapy data processing computing system 110 may be connected to a network (not shown), and such network may be connected to the Internet. For instance, a network can connect the radiotherapy data processing computing system 110 with one or more private and/or public medical information sources (e.g., a radiology information system (RIS), a medical record system (e.g., an electronic medical record (EMR)/ electronic health record (EHR) system), an oncology information system (OIS)), one or more image data sources, an image acquisition device (e.g., an imaging modality). In the depicted example, the radiotherapy data processing computing system 110 is operably coupled to a treatment planning data source 150 (e.g., a database that stores treatment plans) and a treatment device 160 (e.g., a radiation therapy device that implements the treatment plans).

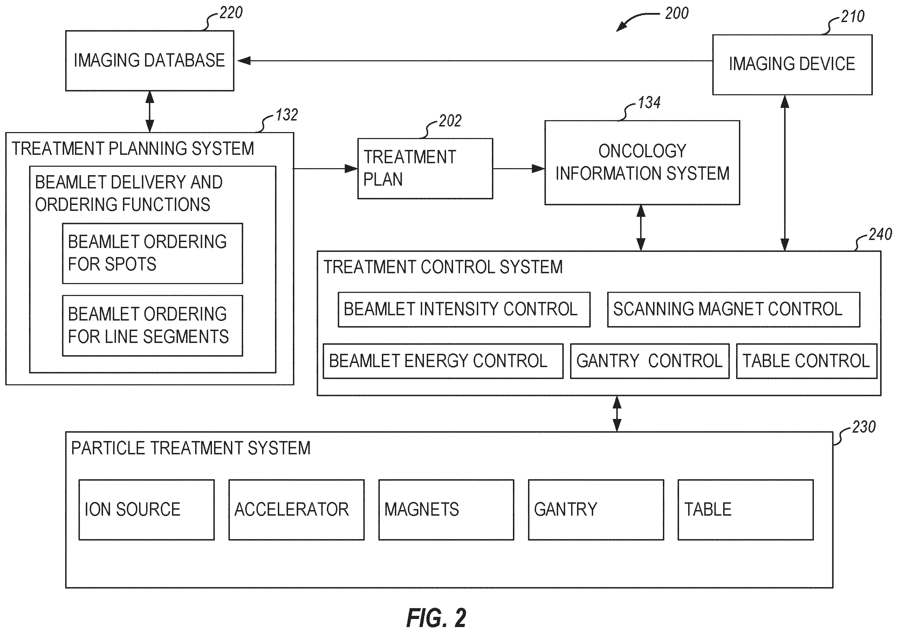

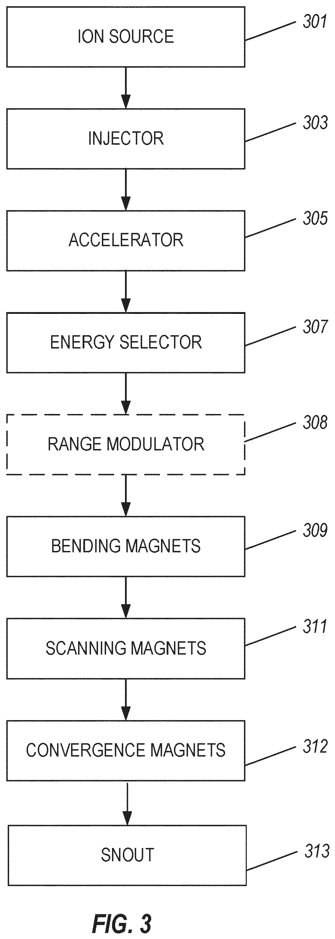

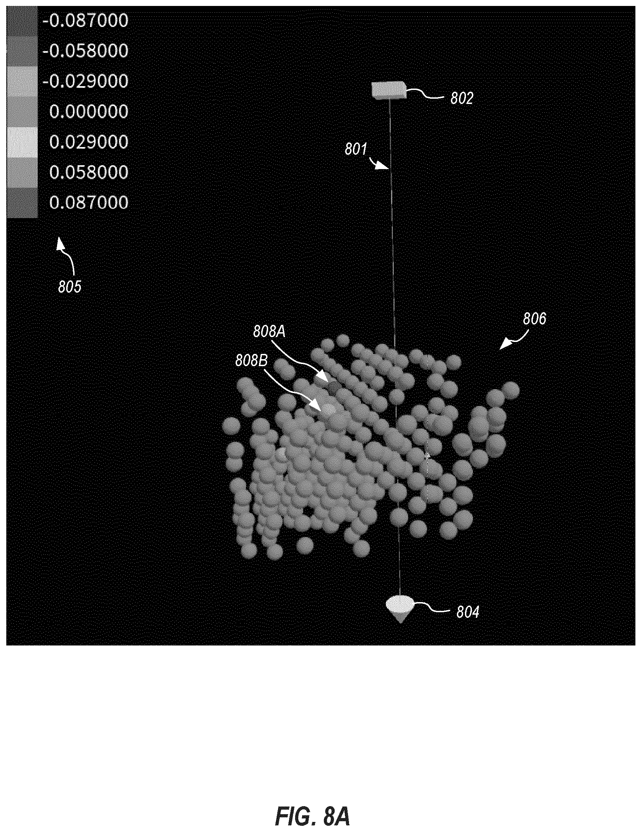

As an example, the radiotherapy data processing computing system 110 can be configured to receive a treatment goal of a subject (e.g., anatomical areas or objects to deliver treatment) and generate a radiotherapy treatment plan by executing instructions or data within a treatment planning system 132, as part of operations to generate treatment plans to be used by the treatment device 160 and/or output on the output device 142. In an embodiment, the treatment planning system 132 is a software application or computer platform that includes programmed functionality to generate, validate, and optimize a radiotherapy treatment plan for each patient. Other information systems include an oncology information system 134 (discussed in more detail with reference to FIG. 2), a treatment control system 136 (discussed in more detail with reference to FIG. 2 and operations of FIGS. 3-7), and a spot delivery visualization system 138 (discussed in more detail with reference to FIGS. 8A to 12).

The radiotherapy data processing computing system 110 may include processing circuitry 112, memory 114, a storage device 116, and other hardware and software-operable features such as a user interface 140, a communication interface (not shown), and the like. The storage device 116 may store transitory or non-transitory computer-executable instructions, such as an operating system, radiation therapy treatment plans, training data, software programs (e.g., image processing software, image or anatomical visualization software, artificial intelligence (AI) or ML implementations and algorithms such as provided by deep learning models, ML models, and neural networks (NNs), etc.), and any other computer-executable instructions to be executed by the processing circuitry 112.

In an example, the processing circuitry 112 may include at least one processing device, such as one or more general-purpose processing devices such as a microprocessor, a central processing unit (CPU), a graphics processing unit (GPU), an accelerated processing unit (APU), or the like. More particularly, the processing circuitry 112 may be a complex instruction set computing (CISC) microprocessor, a reduced instruction set computing (RISC) microprocessor, a very long instruction Word (VLIW) microprocessor, a processor implementing other instruction sets, or processors implementing a combination of instruction sets. The processing circuitry 112 may also be implemented by one or more special-purpose processing devices such as an application-specific integrated circuit (ASIC), a field programmable gate array (FPGA), a digital signal processor (DSP), a System on a Chip (SoC), or the like.

As would be appreciated by those skilled in the art, in some examples, the processing circuitry 112 may be a special-purpose processor rather than a general-purpose processor. The processing circuitry 112 may include one or more known processing devices, such as a microprocessor from the Pentium^{™}, Core^{™}, Xeon^{™}, or Itanium^{™} family manufactured by Intel^{®}, the Turion^{™}, Athlon^{™}, Sempron^{™}, Opteron^{™}, FX^{™}, Phenom^{™} family manufactured by AMD^{™}, or any of various processors manufactured by Sun Microsystems. The processing circuitry 112 may also include graphical processing units such as a GPU device provided from the GeForce^{®}, Quadro^{®}, Tesla^{®} family manufactured by Nvidia^{™}, GMA, Arc^{™} family manufactured by Intel^{®}, or the Radeon^{™} family manufactured by AMD^{®}. The processing circuitry 112 may also include accelerated processing units such as those incorporated into the Xeon^{™} family manufactured by Intel^{®}.

In some examples, the processing circuitry 112 may include or be arranged into a parallel processing configuration. For instance, a set of graphical processing units (e.g., GPU cores, units, devices, or cards) may be arranged to perform highly parallel or repetitive computing tasks simultaneously. The disclosed embodiments are not limited to any type of processor(s) otherwise configured to meet the computing demands of identifying, analyzing, maintaining, generating, and/or providing large amounts of data or manipulating such data to perform the methods disclosed herein. In addition, the term "processor" may include more than one physical (circuitry-based) or software-based processor (for example, a multi-core design or a plurality of processors each having a multi-core design). The processing circuitry 112 can execute sequences of transitory or non-transitory computer program instructions, stored in memory 114, and accessed from the storage device 116, to perform various operations, processes, and methods that will be explained in greater detail below. It should be understood that any component in the radiotherapy system 100 may be implemented separately and operate as an independent device and may be coupled to any other component in the radiotherapy system 100 to perform the techniques described in this disclosure.

The memory 114 may comprise read-only memory (ROM), a phase-change random access memory (PRAM), a static random access memory (SRAM), a flash memory, a random access memory (RAM), a dynamic random access memory (DRAM) such as synchronous DRAM (SDRAM), an electrically erasable programmable read-only memory (EEPROM), a static memory (e.g., flash memory, flash disk, static random access memory) as well as other types of random access memories, a cache, a register, a compact disc read-only memory (CD-ROM), a digital versatile disc (DVD) or other optical storage, a cassette tape, other magnetic storage device, or any other non-transitory medium that may be used to store information including images, training data, one or more ML model(s) or technique(s) parameters, data, or transitory or non-transitory computer executable instructions (e.g., stored in any format) capable of being accessed by the processing circuitry 112, or any other type of computer device. For instance, the computer program instructions can be accessed by the processing circuitry 112, read from the ROM, or any other suitable memory location, and loaded into the RAM for execution by the processing circuitry 112.

The storage device 116 may constitute a drive unit that includes a transitory or non-transitory machine-readable medium on which is stored one or more sets of transitory or non-transitory instructions and data structures (e.g., software) embodying or utilized by any one or more of the methodologies or functions described herein (including, in various examples, the particle therapy information systems 120 and the user interface 140). The instructions may also reside, completely or at least partially, within the memory 114 and/or within the processing circuitry 112 during execution thereof by the radiotherapy data processing computing system 110, with the memory 114 and the processing circuitry 112 also constituting transitory or non-transitory machine-readable media.

The memory 114 and the storage device 116 may constitute a non-transitory computer-readable medium. For example, the memory 114 and the storage device 116 may store or load transitory or non-transitory instructions for one or more software applications on the computer-readable medium. Software applications stored or loaded with the memory 114 and the storage device 116 may include, for example, an operating system for common computer systems as well as for software-controlled devices. The radiotherapy data processing computing system 110 may also operate a variety of software programs comprising software code for implementing the treatment planning system 132, the oncology information system 134, the treatment control system 136, and the spot delivery visualization system 138. Further, the memory 114 and the storage device 116 may store or load an entire software application, part of a software application, or code or data that is associated with a software application, which is executable by the processing circuitry 112.

As non-limiting examples, the memory 114 and the storage device 116 may store, load, and manipulate one or more radiation therapy treatment plans, pre- or post-treatment visualizations, imaging data, segmentation data, histograms or measurements, one or more AI model data (e.g., weights and parameters of one or more ML model(s)), training data, labels and mapping data, and the like. It is contemplated that software programs may be stored not only on the storage device 116 and the memory 114 but also on a removable computer medium, such as a hard drive, a computer disk, an optical disk, USB flash drive, an SD card, a memory stick, or any other suitable medium; such software programs may also be communicated or received over a network.

Although not depicted, the radiotherapy data processing computing system 110 may include a communication interface, network interface card, and communications circuitry. An example communication interface may include, for example, a network adaptor, a cable connector, a serial connector, a USB connector, a parallel connector, a high-speed data transmission adaptor (e.g., such as fiber, USB 3.0, thunderbolt, and the like), a wireless network adaptor (e.g., such as an IEEE 802.11/Wi-Fi adapter), a telecommunication adapter (e.g., to communicate with 3G, 4G/LTE, and 5G networks and the like), and the like. Such a communication interface may include one or more digital and/or analog communication devices that permit a machine to communicate with other machines and devices, such as remotely located components, via a network. The network may provide the functionality of a local area network (LAN), a wireless network, a cloud computing environment (e.g., software as a service, platform as a service, infrastructure as a service, etc.), a client-server, a wide area network (WAN), and the like. For example, the network may be a LAN or a WAN that may include other systems (including additional image processing computing systems or image-based components associated with medical imaging or radiotherapy operations).

Components of the radiotherapy system 100 may be implemented as a virtual machine (e.g., via VMWare, Hyper-V, and the like virtualization platforms) or independent devices. For instance, a virtual machine can be software that functions as hardware. Therefore, a virtual machine can include at least one or more virtual processors, one or more virtual memories, and one or more virtual communication interfaces that together function as hardware. For example, the radiotherapy data processing computing system 110, the image data sources, or like components, may be implemented as a virtual machine or within a cloud-based virtualization environment.

The processing circuitry 112 may be communicatively coupled to the memory 114 and the storage device 116, as the processing circuitry 112 is configured to execute computer-executable instructions stored thereon from either the memory 114 or the storage device 116. Particularly, spot delivery visualization system 138 is adapted to implement operations, outputs, and other workflow actions for the presentation of information from radiotherapy treatment, for the visualization of a particle beam therapy (e.g., such as the graphical output depicted in FIGS. 8A to 11C). The oncology information system 134 can communicate information to the spot delivery visualization system 138, or the spot delivery visualization system 138 may be implemented as functionality or a sub-system within the oncology information system 134 to visualize particle therapy outcomes measured with the oncology information system 134.

The radiotherapy data processing computing system 110 may communicate with an external database (e.g., hospital database, medical records system) through a network to send/receive a plurality of various types of data related to medical and radiotherapy operations. The external database may be a storage device and may be equipped with appropriate database administration software programs. Further, such databases or data sources may include a plurality of devices or systems located either in a central or a distributed manner. The radiotherapy data processing computing system 110 can collect and obtain data, and communicate with other systems, via a network using one or more communication interfaces, which are communicatively coupled to the processing circuitry 112 and the memory 114. For instance, a communication interface may provide communication connections between the radiotherapy data processing computing system 110 and radiotherapy system components (e.g., permitting the exchange of data with external devices).

The radiotherapy data processing computing system 110 may, in various examples, have appropriate interfacing circuitry from an output device 142 or an input device 144 to connect to the user interface 140, which may be a hardware keyboard, a keypad, or a touch screen through which a user may input information or controls into the radiotherapy system. The user interface 140, the output device 142, and the input device 144 may also provide output functions in connection with the spot delivery visualization system 138, including to provide visualizations of particle therapy outcomes. As an example, the output device 142 may include a display device that outputs a representation of the user interface 140 and one or more aspects, visualizations, or representations of the medical images, the treatment plans, and visualizations and information relevant to the deviation of planned versus delivered particle beam therapies at respective spots, areas, or groups of spots and areas. The output device 142 may include one or more display screens that display these visualizations, optionally in addition to medical images, treatment planning parameters (e.g., contours, dosages, beam angles, labels, maps, etc.), treatment plans, a target, or any related information to the user. The input device 144 connected to the user interface 140 may be a keyboard, a keypad, a touch screen, or any type of device that a user may use to the radiotherapy system 100. Alternatively, the output device 142, the input device 144, and features of the user interface 140 may be integrated into a single device such as a smartphone or tablet computer (e.g., Apple iPad^{®}, Lenovo Thinkpad^{®}, Samsung Galaxy^{®}, etc.).

**FIG. 2** illustrates generally an example of a radiation therapy system 200, such as may include a particle treatment system 230 and an imaging device 210, in accordance with an example. The particle treatment system 230 includes an ion source, an accelerator, scanning magnets, and convergence magnets each of which is described in more detail below with respect to FIG. 3. The particle treatment system 230 includes a gantry and a table, where the gantry may be mounted on the table, affixed to the table, or stabilized with respect to the table. The table may hold a patient. The gantry may be a rotating gantry, and may rotate with respect to the table (e.g., around the table) or with respect to the patient (and the table or a portion of the table may rotate with the gantry).

The particle treatment system 230 may communicate with a treatment control system 240, which may be used to control actions of the particle treatment system 230. The treatment control system 240 may communicate with an imaging device 210 (e.g., to receive images taken by an imaging acquisition device or an imaging database) or the oncology information system 134. The oncology information system 134 may provide a treatment plan 202 to the treatment control system 240, such as received from the treatment planning system 132. The treatment control system 240 may use the treatment plan 202 to control the particle treatment system 230 (e.g., activate the gantry, the ion source, the accelerator, the scanning magnets, the convergence magnets, a particle beam, or the like). The treatment control system 240, for example, may include a beamlet intensity control, a beamlet energy control, a scanning magnet control, a convergence magnet control, a table control, a gantry control, etc. In an example, the beamlet intensity control and the beamlet energy control may be used to activate a beamlet of a particular size or to target a particular location. The scanning magnetic control or the convergence magnet control may be used to deliver beamlets according to the treatment plan 202, for example in a convergence pattern. In some examples, the scanning magnet control and the convergence magnet control may be a single control. The gantry control or the table control may be used to rotate the gantry.

The treatment planning system 132 may include components such as a beamlet delivery and ordering functions, with, for example, separate controls for beamlet ordering for spots or line segments. The treatment planning system 132 may include spot delivery or beamlet functions configured to plan the size of beamlets, location of a target or spot, or the like. The beamlet functions may be used to determine an order of delivery of beamlets, for example in a spiral pattern. The order of delivery functions may be in communication with the treatment planning system 132 for planning delivery of beamlets to particular spots or areas of treatment. Accordingly, the treatment planning system 132 may be used to determine or plan a gantry angle, gantry speed, beamlet size, spiral pattern (e.g., clockwise or counterclockwise), angle range for a particular spiral pattern (e.g., every ten degrees of the gantry rotation), or the like.

Additionally, the treatment planning system 132 may access an imaging database 220 to retrieve images or store information. When the treatment plan 202 is completed, the treatment planning system 132 may send the treatment plan 202 to the oncology information system 134 for communication with the treatment control system 240.

**FIG. 3** illustrates an example operational flow of a particle treatment system 300 that may include a radiation therapy output configured to provide a proton therapy beam. The particle treatment system 300 includes an ion source 301, an injector 303, an accelerator 305, an energy selector 307, a plurality of bending magnets 309, a plurality of scanning magnets 311, a plurality of convergence magnets 312, and a snout 313. The plurality of convergence magnets 312 may be used to converge a beam initiated by the ion source 301, in some examples.

The ion source 301, such as a synchrotron (not shown) may be configured to provide a stream of particles, such as protons. In an example, the stream of particles is transported to an injector 303 that provides the charged particles with an initial acceleration using a Coulomb force. The particles are further accelerated by the accelerator 305 to about 10% of the speed of light. The acceleration provides energy to the particles, which determines the depth within tissue the particles may travel. The energy selector 307 (e.g., a range scatter) may be used to select the energies of the particles to be delivered to the patient. In an example called passive scattering, an optional range modulator 308 (e.g., also called a ridge filter or a range modulation wheel) may be utilized to broaden the beam to fit the tumor. After selecting energies, a plurality of bending magnets 309 may be utilized to transport the stream of particles into a radiation therapy treatment room. Further, scanning magnets 311 (e.g., x-y magnets) are used to spread the particle beam to, or trace, an exact image of the tumor shape. A snout 313 is used to further shape the particle beam. In various examples, the stream of particles may be composed of protons, carbon ions, pions, positively charged ions, or the like.

After the scanning magnets 311 spread the particle beam, the particle beam may be in a diverged state (e.g., outline of the delivery pattern on the skin at entrance to the patient is smaller than the outline at the target within the patient). The convergence magnets 312 may be used to converge the particle beam from the diverged state to a converging state. The converging state particle beam may be configured using the convergence magnets 312 to converge at a target (e.g., at a tumor or past a tumor in a patient).

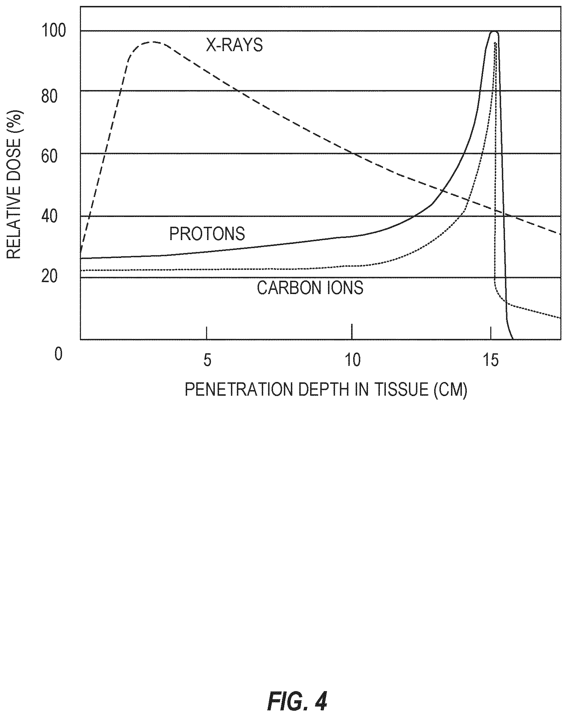

**FIG. 4** provides an illustration of a comparison of radiation dose depths for various types of particles in human tissue. As shown, the relative depth of penetration into human tissue of photons (e.g., x-rays) versus protons versus carbon ions is provided (e.g., including any radiation dose provided at a distance beneath the surface, including secondary radiation or scatter). Each radiation dose is shown relative to the peak dose for a proton beam having a single energy which has been set to 100%.

The mono-energetic (e.g., single energy) proton beam indicates a plateau region starting at approximately 25% that gradually increases until approximately 10 cm depth in tissue where it rapidly increases to the Bragg Peak at 15cm and then advantageously falls to zero within a short distance. No additional dose is delivered at the end of the Bragg peak.

The photon beam (e.g., labeled as X-rays) indicates the initial build up due to electron scatter (e.g., the primary means by which X-rays deliver dose to tissue is through transfer of energy to electrons in the tissue). This is followed by an exponential fall off, which continues past the distal edge of the target, which is at approximately 15 cm depth in the diagram. The x-ray beam has an entrance (skin) dose set to match that of the proton beam. With normalization (e.g., scaling) at 15 cm depth, the dose due to x-rays is at 40% of the dose provided by proton beam, while the x-ray beam has a peak dose of greater than 95% ("near" 100%) at approximately 3cm depth. If the x-ray data is renormalized to achieve 100% dose at 15 cm, the peak dose at approximately 3cm depth would be approximately 240%, in a location where dose is not desired (e.g., prior to the target). Therefore, with x-rays, a considerable amount of dose is delivered prior to the target and an appreciable amount of dose is delivered past the target.

The mono-energetic carbon ion beam shows a plateau region at the entrance dose that is lower than the proton beam. The carbon ion beam has a sharper Bragg Peak that falls more precipitously than the proton beam, but the carbon ion beam has a tail (e.g., known as a "spallation tail", where some of the Carbon nuclei shatter into Helium ions) that has approximately 10% additional dose, or less, past the desired target by several centimeters. The carbon ion beam has an undesired entrance and skin dose compared to the proton beam, but the carbon ion beam has a non-trivial dose delivered past the target.

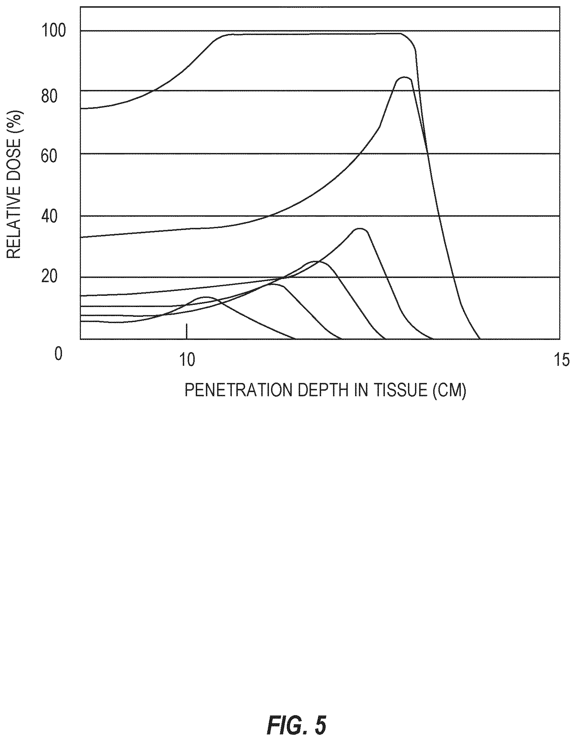

**FIG. 5** provides an illustration of a spread-out Bragg peak (SOBP). The SOBP. displays a relative depth dose curve for the combination of a set of proton beams of various initial energies each of which has had some spread in energy (e.g., variable absorption of energy in tissue). The desired result of having a uniform dose for a target of a particular thickness. As shown, the target is shown with a proximal depth of approximately 10 cm, a distal depth of approximately 13 cm, and a target thickness of approximately 3 cm. Within the target, the dose is quite uniform (with an average normalized at 100%). The diagram does not start at 0 cm depth and is not explicitly showing the entrance (skin) dose, but the nature of the entrance region of proton beams is a relatively flat depth dose curve. Typically, the entrance (skin) dose will be approximately 70% of the target dose (e.g., shown at the far right edge of the x-axis). An SOBP may be obtained using a variety of approaches, including using a scattered proton beam with modulation of the energy (variable absorption) utilizing a variety of devices (e.g., a static ridge filter or a dynamic range modulation wheel), or by selection of a number of mono-energetic proton beams that do not undergo scatter.

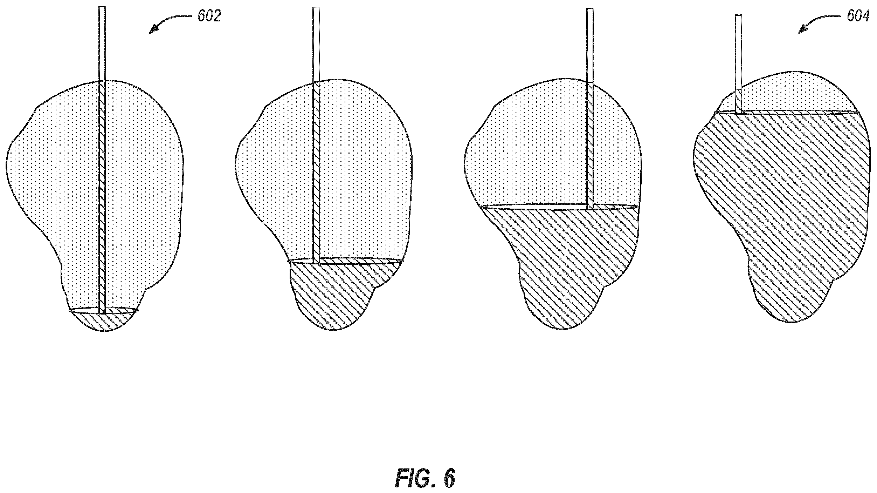

**FIG. 6** provides an illustration of a Pencil Beam Scanning of an irregular shape volume from a distal edge (e.g., bottom) to a proximal (e.g., top) edge. As shown, the irregular shaped tumor volume is irradiated layers of protons. For example, a first time snapshot 602 shows a first layer of protons being delivered, and a later time snapshot 604 shows that most of the layers have been delivered. Each layer has its own cross-sectional area to which the protons having the same energy are delivered. The total radiation dose is provided as a layer-by-layer set of beamlets. Each layer of may have different energies. The most common means of specifying and delivering the set of beamlets to the cross-sectional area is to define and deliver beamlets having a constant diameter ("spot size") to a selection of grid points on each layer. While the majority of the dose from the beamlet is delivered to the targeted layer, a significant amount of dose is delivered along the path to the targeted layer. The dose to proximal layers from beamlets defined for distal layers is accounted for in the specification of the beamlets defined for the proximal layers. The ability to individually specify the number of particles (e.g., the meterset) for a given beamlet ensures that each part of the volume being irradiated receives the desired dose.

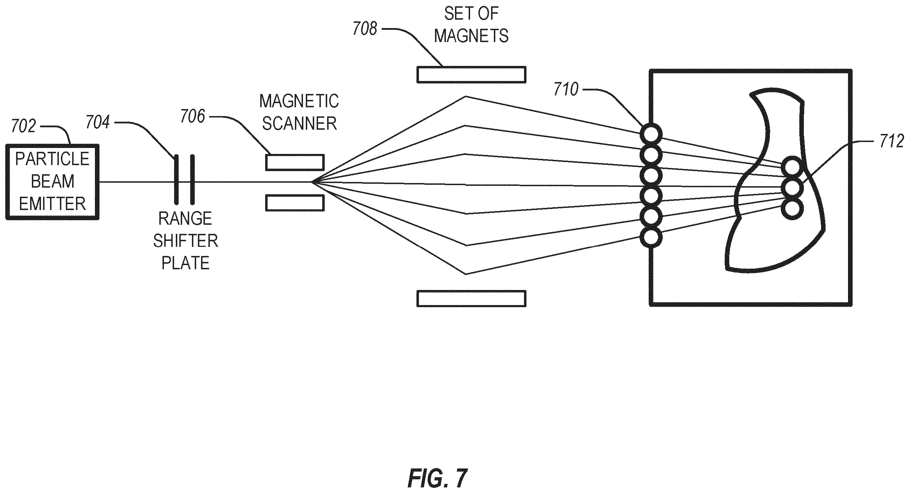

**FIG. 7** provides an illustration of a diagrammatic representation of an example active scanning proton beam delivery system configured to deliver a convergent particle beam. As shown, a particle beam is emitted by a particle beam emitter 702. An incoming mono-energetic proton beamlet has a specified amount of its energy absorbed by the range shifter (e.g., a range shifter plate 704), resulting in a beamlet with the desired energy to achieve a certain depth for the Bragg Peak in the patient to treat the specified layer. A magnetic scanner 706, which has the ability to deflect the particles in both a vertical and a horizontal direction may be used to diverge the particle beam. The strength of the magnetic fields in the magnetic scanner 706 may be adjusted to control the deflection in the direction perpendicular to the magnetic field and the incoming beamlet. The rate at which the magnetic field strengths may be adjusted determines the rate at which the scanning may take place. For example, the intensity of the proton beamlet in combination with the scanning rate determines how much dose may be delivered to a specific area in a particular amount of time (e.g., particles/unit area).

After diverging via the magnetic scanner 706, a set of magnets 708 may be used to converge the divergent beam such that the beam converges at a target 712 in a patient. A spread width (e.g., size) of delivery may occur at a skin entry area 710 of the patient that is larger than a converged spread width at the target 712. By diverging and then converging the beam via the magnetic scanner 706 and a set of magnets 708, radiation delivered to the skin entry area 710 can be more spread out than that delivered to the target 712. This may provide a lower dosage per area unit on the skin of the patient than at the target. This may provide an opportunity to deliver a higher dose to the target 712 with a same skin radiation delivery (e.g., if the beam was divergent), which may allow for fewer factions to be needed. In some examples, instead of delivering a higher dose to the target 712, a lower dose may be delivered per area unit on the skin while maintaining a same dose to the target, reducing the severity of side effects to the skin.

The planning of particles with pencil beam therapy can be represented with a variety of three-dimensional visualizations. One such example, before treatment, is the use of the treatment planning system 132 to present a visualization of the field of therapy using dots or spots that represent the delivery of locations (and optionally, amounts) of radiation planned to be delivered to target 712. These dots can be arranged within a field that portrays a 3D shape of the treatment delivery, corresponding to the grid points discussed above, which is presented to a user via a 2D interface such as a computer screen.

The actual delivery of particles with the pencil beam therapy can also be represented with dots or spots after treatment, using the spot delivery visualization system 138. These dots are illustrated in respective 3D views in the various examples of **FIGS. 8A** to **11C****,** and discussed in more detail below. As will be understood, individual dots represent a particular sample point and will be reduced from their actual size (e.g., shrunk down) to portray a human-understandable representation of the radiation that has been actually delivered in a particular area. Thus, in a visualization of particle therapy, a respective dot represents one of the many volumetric (e.g., square or rectangular) three-dimensional areas exposed to the particle beam treatment. A combination of dots, established from the exposure to many particle beams, corresponds to the area of the treatment for the tumor.

In a visualization of a planned treatment field, an arrangement of dots can be used to represent the amount and location of planned radiation. This concept is extended with the approaches below to present a dose delivery visualization, with dots that portray a deviation in the actual amount of delivered radiation relative to the planned amount of radiation (and, where applicable, deviation relative to the planned location of radiation). This visualization of delivered therapy enables a user to identify dose or dose position discrepancies of the delivered therapy relative to a treatment plan. In an interactive user interface, the visualization can be presented to a user that allows the user to zoom out and obtain an overall sense of the delivered dose shape, or allow the user to zoom in to inspect individual spots and experienced discrepancies.

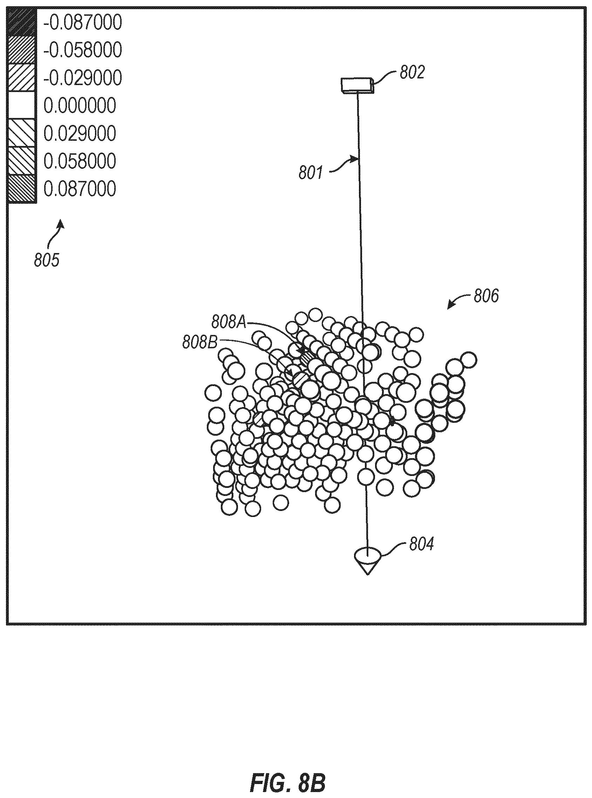

**FIG. 8A** and **FIG. 8B** depict a representation of treatment spots and dose deviation provided by particle therapy. As explained above, the spot delivery visualization system 138 can provide a dose delivery visualization based on treatment data from the oncology information system 134. This dose delivery visualization may provide a 3D representation of the delta (e.g., difference or deviation) between the planned arrangement of treatment spots versus the arrangement of treatment spots that were delivered.

**FIG. 8A** first depicts a color view of the dose delivery visualization, in a perspective view of a visual array of treatment spots 806. The visual array is generated by comparing spot information from the treatment plan and the treatment record from the delivery device. Each treatment spot is represented in the visualization by a 3D sphere (e.g., circular dot). The overall dose shape in the treatment area is thus represented by clusters of these individual 3D spheres, such as may correspond to the shape of a tumor. Changes in the colors of the individual 3D spheres can be used to designate no deviation, deviation, and an amount of deviation at each treatment spot.

In an example, dose discrepancies are displayed by a color mapping 805, with different colors, shades, or patterns being designated based on some range or pre-defined/associated value(s) of dose deviation. For example, a spot that has been delivered with the correct dose (e.g., within a range associated with the correct planned dose) will be represented as green. Over-delivery of a spot is represented within a progressive color scale, along a yellow, orange, and red color spectrum. Under-delivery of a spot is also represented with a progressive color scale, along a light blue, blue, and purple color spectrum. Each of these defined colors of the color mapping 805 can correspond to ranges of dose deviation (with median numerical values depicted). In the depiction of FIG. 8A, a majority of the treatment spots 806 have been delivered correctly (depicted in green), with two spots slightly underdelivered (depicted in light blue, including spot 808B) and one spot over-delivered (depicted red, including spot 808A). The pointer 804 shows the direction of the beam 801, with the rectangle 802 representing the delivery gantry.

**FIG. 8B** depicts a black-and-white line drawing representation of the view of FIG. 8A. Here, shading and patterns are used in place of colors. A variety of visual effects may be used to indicate over-delivery (more delivered radiation dose than planned), correct delivery (no dose deviation), and under-delivery (less delivered radiation dose than planned). Accordingly, other effects to demonstrate dose delivery deviation may involve aspects of shading, patterns or fills, vibrancy (e.g., brightness or contrast), transparency or opacity, and the like.

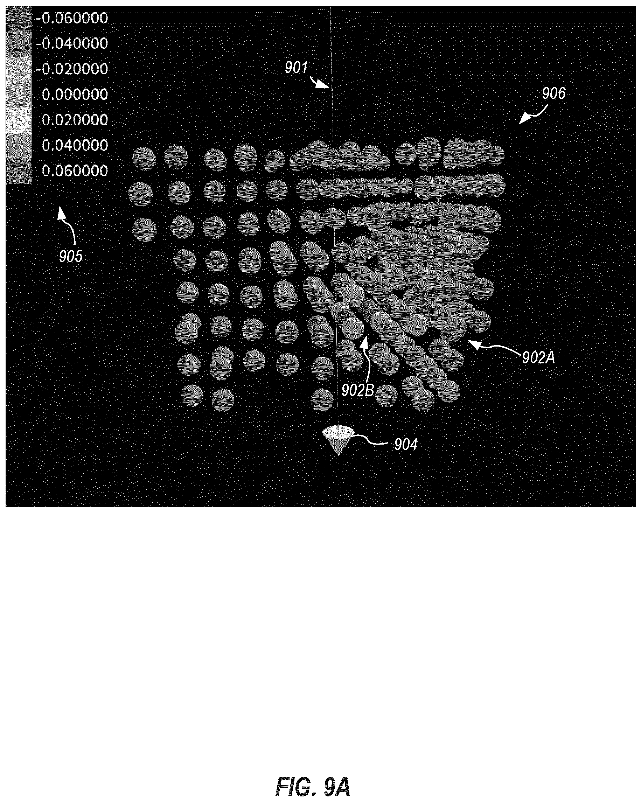

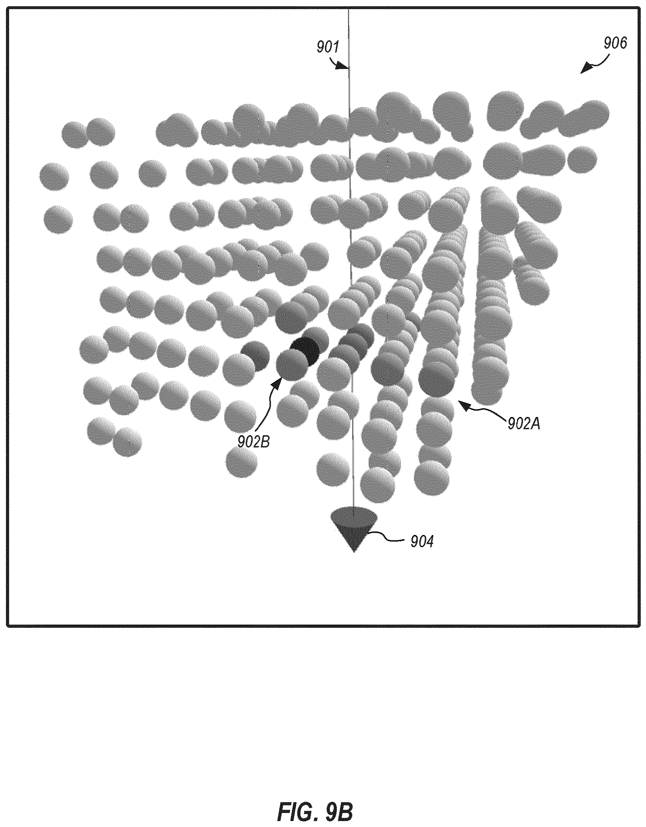

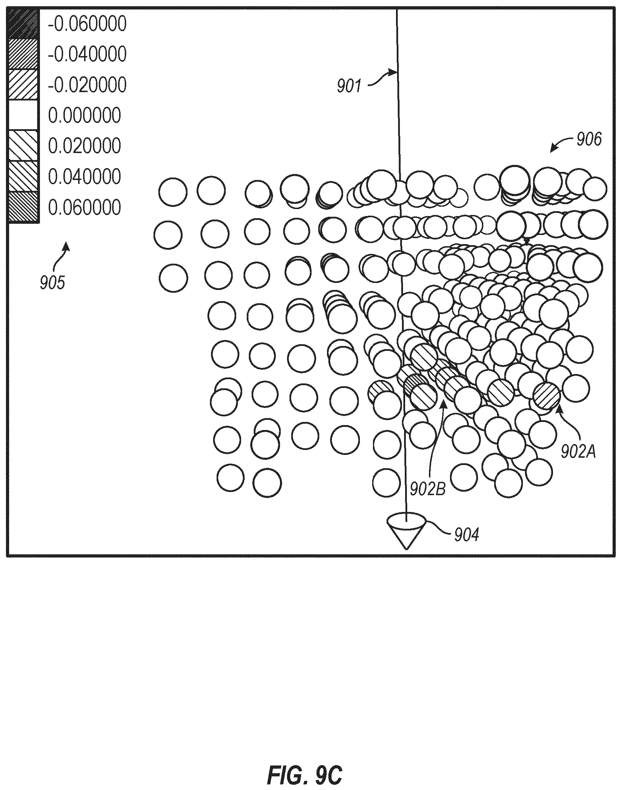

**FIGS. 9A** to **9C** depict another representation of treatment spots and dose deviation provided by particle therapy, from a front view. **FIG. 9A** depicts a color representation of the dose delivery visualization; **FIG. 9B** depicts the view of FIG. 9A, in grayscale; and **FIG. 9C** depicts the view of FIG. 9A in a black-and-white line drawing representation.

In FIG. 9A, similar to the description of FIG. 8A above, a color mapping 905 provides a progressive color scale to indicate a spot that has been delivered with the correct dose (e.g., within a predetermined range associated with the correct planned dose) as green. Other colors are used to represent over-delivery (e.g., yellow, orange, red) or the under-delivery (e.g., light blue, dark blue purple) of a dose. The pointer 904 shows the direction of the beam 901. Within the depiction of FIG. 9A, a majority of the treatment spots 906 are green and have been correctly delivered according to the treatment plan. One spot 902A designates a spot that has been under-delivered relative to the treatment plan. Another set of spots 902B indicates a spot that has been over-delivered, with nine spots having respective yellow, orange, and red colors.

In FIG. 9B, a grayscale representation with grayscale shading is used in place of colors. Likewise in FIG. 9C, a black-and-white line drawing with shading and patterns is used in place of colors. As discussed above, a variety of visual effects may correspond to particular ranges and values that represent dose deviation (and non-deviation or range conformance). In some black-and-white or grayscale examples, the amount of shading corresponds to the amount of deviation-whether positive or negative deviation.

In further examples, dose position discrepancies can be represented. These position discrepancies can be displayed with an arrow or other directional indicator at or adjoining each spot, e.g., an adjacent arrow pointing in the direction of the discrepancy. Other characteristics such as the length of the arrow can be used to represent the amount or type of directional discrepancy.

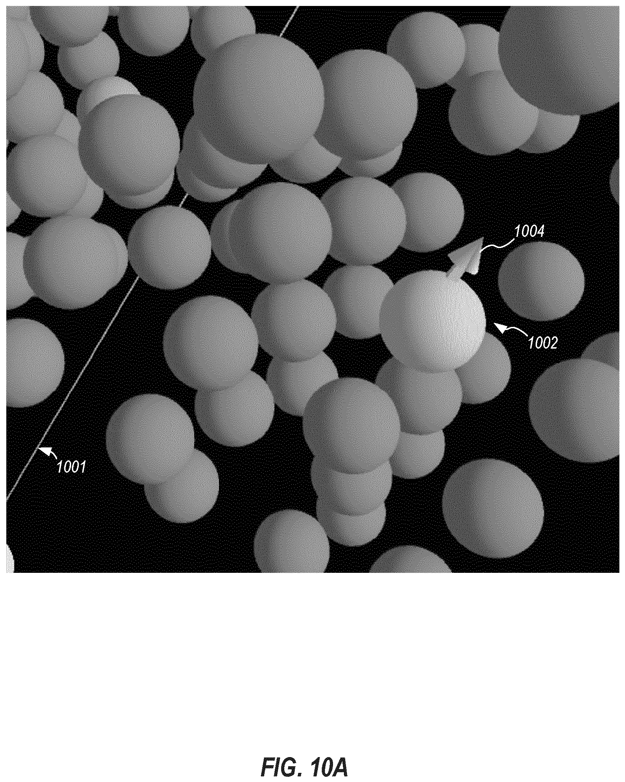

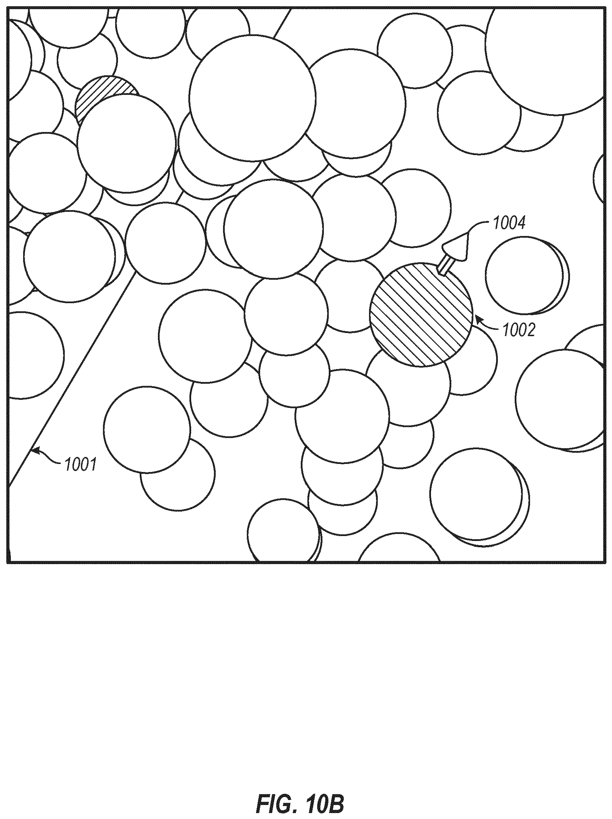

**FIGS. 10A** and **10B** depict a representation of treatment spots and dose position deviation provided by particle therapy, from a perspective close-up view of treatment spots. FIG. 10A depicts a color representation of a beam 1001 and a field of dots surrounding this beam. A particular spot 1002 has a yellow color to indicate that the treatment was over-delivered at the position corresponding to the particular spot 1002. An arrow 1004 is attached to the particular spot 1002, to indicate that deviation in the indicated direction. In other words, the dose was over-delivered at the particular spot 1002, and the dose should have been delivered in the direction of the arrow 1004.

FIG. 10B depicts a black-and-white line drawing representation of FIG. 10A. Here, shading is used in place of colors. As depicted in FIG. 10A, the position discrepancy on each spot is represented by an arrow, showing both the direction and amount of the discrepancy.

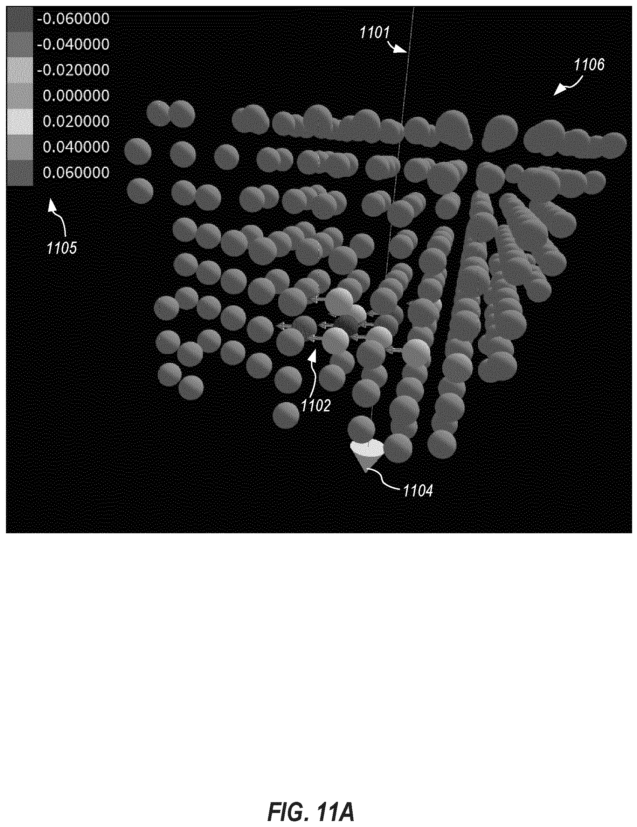

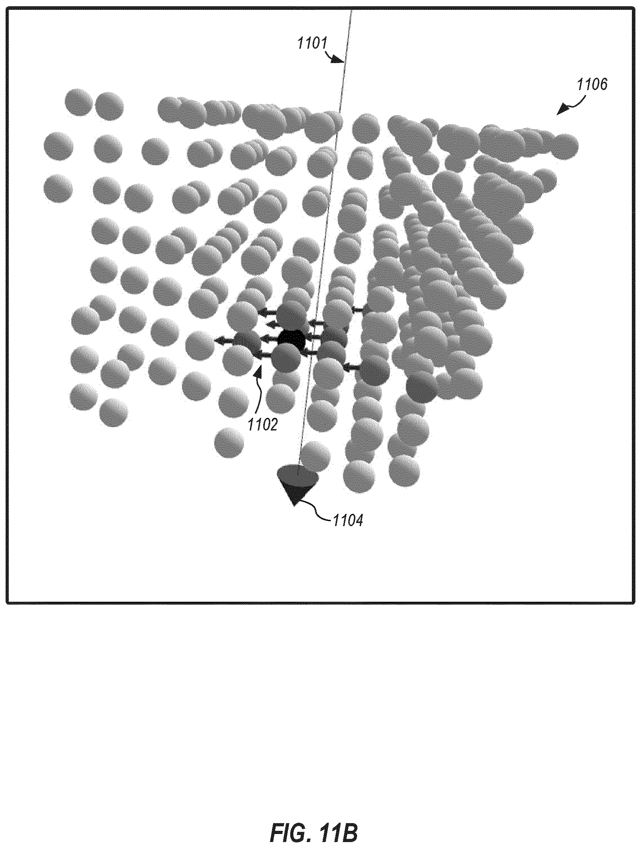

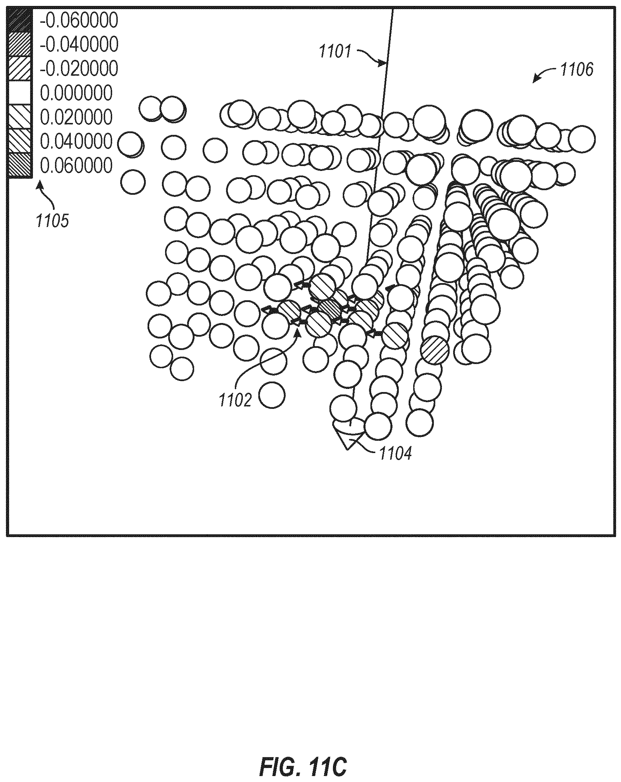

**FIG. 11A****,** **FIG. 11B****,** and **FIG. 11C** depict other representations of treatment spots and dose location deviation provided by a particle therapy. These representations show treatment spots 1106 resulting from doses of a beam 1001 that was provided in a direction of a pointer 1104. These representations include a color mapping 1105, similar to those described for FIGS. 8A to 9C above.

FIG. 11A first depicts a color representation with a group of deviating treatment spots 1102, in the treatment spots 1106, which have experienced an excess delivered dose, as compared with the planned dose. The deviating treatment spots 1102 include spots depicting different levels of deviation, designated with different colors red, orange, and yellow. However, the deviating treatment spots 1102 includes directional arrows of the same size and direction, showing that deviation of the dose delivery uniformly occurred in this area (and, the dose was supposed to be delivered in a direction that the arrow is pointing).

FIG. 11B depicts the view of FIG. 11A, in a grayscale representation. Here, this visualization uses grayscale shades and arrows to show that the deviating treatment spots 1102 experienced some position discrepancy and were over-delivered at those respective locations. FIG. 11C depicts a black-and-white line drawing representation of FIG. 11A.

As will be understood, the visualizations of FIGS. 8A to 11C represent a small field of treatment, such as would correspond to planned and delivered treatment for a very small tumor. For larger tumors, many more spots would be used to represent the particle therapy treatment spots or areas. Because a representation of a large tumor would be overwhelming, with potentially thousands of spots, a variety of display adaptation techniques may be used to simplify the view. Various aspects of a user interface may allow exclusion of certain spots, inclusion of certain spots, or filtering to be applied to the field or the dots, based on some criterion/criteria that allows dose deviation and dose position deviations to be more clearly illustrated.

The improvements of the spot delivery visualization described herein can be distinguished from existing approaches used in an OIS or a treatment planning system. Although an OIS can record detailed information about a particle beam treatment, an OIS only includes information to identify if there is a discrepancy between planned vs. delivered dose at the treatment field level, and not at the spot level. Likewise, although an OIS could be configured to alert a user that a certain spot was out of tolerance for dose or position, based on raw information such as "spot # 452 was x% out of tolerance", this type of information does not provide the appropriate context to make a clinical decision. The visualizations as discussed herein can thus provide a new form of information and user guidance that is not available.

The spot delivery visualization techniques can apply to multiple types of particle therapy treatments and are not limited to current implementations of pencil beam scanning. Thus, the spot delivery visualization techniques can work for a variety of shapes of tumors and treatment areas. Additional user interface functionality (not specifically discussed above) may be coordinated with the spot delivery visualization to allow a user to pan, rotate, or manipulate views on demand. As noted above, other variations may include applying filters that could turn on and off to hide certain types of dots/spots; applying transparency; conversion of colors or grayscale settings; hiding or de-emphasizing particular types of dots/spots; use of layers to allow dynamic presentation and removal of certain areas or types of dots/spots; and similar visual effects.

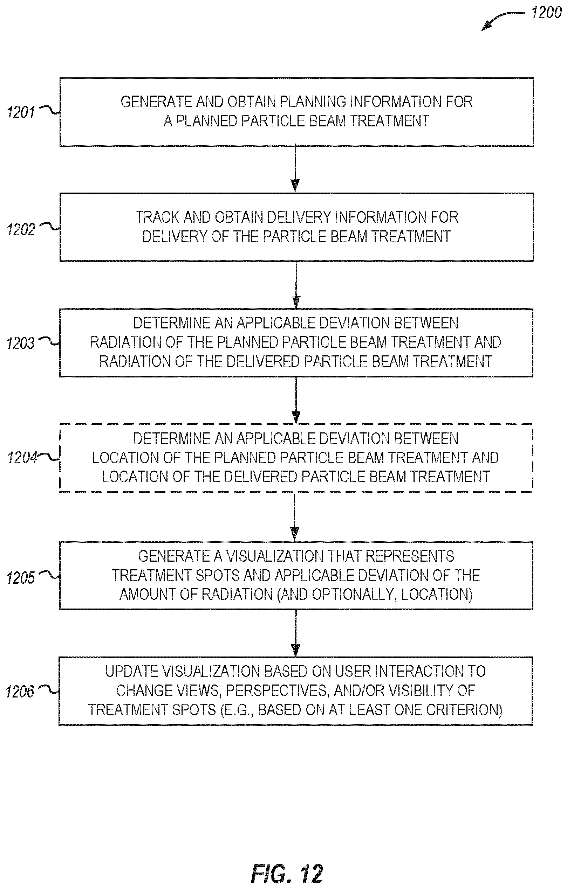

**FIG. 12** is a flowchart 1200 of an example method for visualizing results of a particle beam treatment delivery. This method may include variations based on the visualizations and functionality described with reference to FIGS. 8A to 11C.

At 1201: Operations are performed to generate and obtain planning information for a planned particle beam treatment. This may include the use of a treatment planning system 132, as discussed above. As noted above, such planning information may relate to treatment planned to be delivered to multiple treatment spots located in a treatment area of a patient (e.g., a tumor).

At 1202: Operations are performed to track and obtain delivery information for delivery of the particle beam treatment. This may include the use of an oncology information system 134, which receives treatment delivery information in connection with the treatment control system 136, as discussed above. As noted above, such delivery information may relate to treatment actually delivered to the multiple treatment spots located in the treatment area of the patient.

At 1203: Operations are performed to determine an applicable deviation between a dose of radiation planned for the planned particle beam treatment, and a dose of radiation delivered from the delivered particle beam treatment, for each of the multiple treatment spots located in the treatment area of the patient. Consistent with treatment planning techniques that are used with particle beam therapies, such treatment spots can correspond to discrete areas of the planned particle beam treatment in a treatment space mapped by the treatment planning system.

At 1204: Operations are performed to optionally determine an applicable deviation between a location of the dose of radiation planned for the planned particle beam treatment, and a location of the dose of radiation actually delivered from the delivered particle beam treatment.

At 1205: Operations are performed to generate and present a visualization that represents treatment spots and applicable deviation of the amount of radiation dose (and optionally, applicable deviation of the location of the radiation dose). This visualization may be performed for each of the treatment spots, or a subset of available treatment spots. In an example, the visualization uses different colors or shading among the treatment spots to represent an amount of the applicable dose deviation between the planned amount of radiation and the delivered amount of radiation (e.g. as shown with reference to FIGS. 8A to 11C).

In a further example, the visualization additionally represents the applicable dose location deviation between the planned location and the delivered location, using a directional indicator for respective spots of the treatment spots. For instance, if the applicable dose deviation exceeds a predetermined threshold for a respective spot, the directional indicator is presented adjacent to the respective spot. This directional indicator can point in a direction from the respective spot towards an area of planned delivery.

In a specific example that visualizes the dose deviation with colors, a first color (e.g., green) may be used to represent respective spots of the treatment spots that have no dose deviation within a predetermined range; a second color is used to represent respective spots of the treatment spots that have a dose deviation outside the predetermined range with less delivered radiation than planned radiation; and a third color is used to represent respective spots of the treatment spots that have a dose deviation outside the predetermined range with more delivered radiation than planned radiation.

At 1206: Operations are performed to update the visualization (based on user interaction or other user inputs, settings, etc.) to change views, perspectives, and/or visibility of the treatment spots (e.g., based on at least one criterion). For example, the visualization may be output in a graphical user interface, providing a three-dimensional representation that arranges respective balls or spheres to represent each of the treatment spots. This graphical user interface can also receive user interaction to change views and perspectives of the visualization, on-demand. This graphical user interface can also receive user interaction to change visibility of the respective spheres, based on at least one criterion.

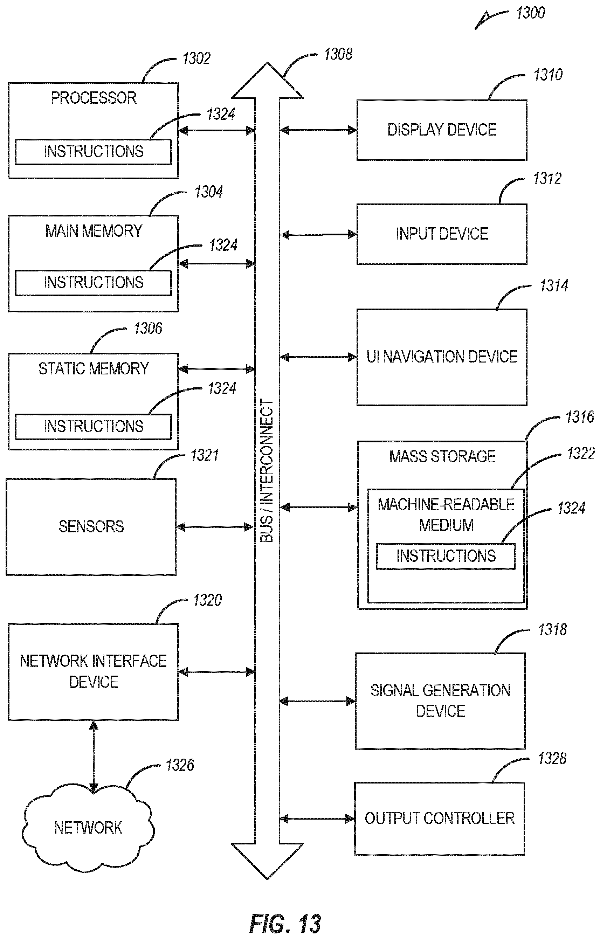

**FIG. 13** is a block diagram of an example of an apparatus, device, system or machine 1300 upon which any one or more of the techniques (e.g., methods) discussed herein may perform. In alternative embodiments, the machine 1300 may operate as a standalone device or may be connected (e.g., networked) to other machines. In a networked deployment, the machine 1300 may operate in the capacity of a server machine, a client machine, or both in server-client network environments. In an example, the machine 1300 may act or operate as a peer machine in peer-to-peer (P2P) (or other distributed) network environment. The machine 1300 may be a personal computer (PC), a tablet PC, server computer, a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

The machine 1300 may receive a user command by way of any of input device 1312, UI navigation device 1314, display device 1310 or microphone (optional) as described herein. The machine 1300 may output an indication that an action has been performed by way of any of display device 1310 and signal generation device 1318. The machine 1300 may output processed medical data in machine readable medium 1322 as described herein. The machine 1300 may be used to implement the operations performed by the spot delivery visualization system 138, and related data processing systems involved in processing and representing particle therapy treatment outcomes and visualizations.

Examples, as described herein, may include, or may operate by, logic or a number of components, or mechanisms. Circuit sets are a collection of circuits implemented in tangible entities that include hardware (e.g., simple circuits, gates, logic, etc.). Circuit set membership may be flexible over time and underlying hardware variability. Circuit sets include members that may, alone or in combination, perform specified operations when operating. In an example, hardware of the circuit set may be immutably designed to carry out or conduct a specific operation (e.g., hardwired). In an example, the hardware of the circuit set may include variably connected physical components (e.g., execution units, transistors, simple circuits, etc.) including a computer readable medium physically modified (e.g., magnetically, electrically, moveable placement of invariant massed particles, etc.) to encode instructions of the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuit set in hardware via the variable connections to carry out or conduct portions of the specific operation when in operation. Accordingly, the computer readable medium is communicatively coupled to the other components of the circuit set member when the device is operating. In an example, any of the physical components may be used in more than one member of more than one circuit set. For example, under operation, execution units may be used in a first circuit of a first circuit set at one point in time and reused by a second circuit in the first circuit set, or by a third circuit in a second circuit set at a different time.

Machine 1300 (e.g., computer system) may include a hardware processor 1302 (e.g., a central processing unit (CPU), a graphics processing unit (GPU), a hardware processor core, field programmable gate array (FPGA), or any combination thereof), a main memory 1304 and a static memory 1306, some or all of which may communicate with each other via an interlink (e.g., bus) 1330. The machine 1300 may further include a display device 1310, an input device 1312 (e.g., a keyboard or other alphanumeric input device), and a user interface UI navigation device 1314 (e.g., a mouse). In an example, the display device 1310, input device 1312, and UI navigation device 1314 may be a touch screen display. Optionally, the machine 1300 includes a microphone (e.g., to receive audio of verbal instructions that are converted to text input). The machine 1300 may additionally include a storage device 1308 (e.g., drive unit or other similar mass storage device or unit), a signal generation device 1318 (e.g., a speaker), a network interface device 1320 connected to a network 1326, and one or more sensors 1321, such as a global positioning system (GPS) sensor, compass, accelerometer, or other sensor. The machine 1300 may include an output controller 1328, such as a serial (e.g., universal serial bus (USB), parallel, or other wired or wireless (e.g., infrared (IR), near field communication (NFC), etc.) connection to communicate or control one or more peripheral devices (e.g., a printer, card reader, etc.).

The storage device 1308 may include a machine readable medium 1322 on which is stored one or more sets of data structures or instructions 1324 (e.g., software) embodying or used by any one or more of the techniques or functions described herein. The instructions 1324 may also reside, completely or at least partially, within the main memory 1304, within static memory 1306, or within the hardware processor 1302 during execution thereof by the machine 1300. In an example, one or any combination of the hardware processor 1302, the main memory 1304, the static memory 1306, or the storage device 1308 may constitute machine readable media.

While the machine readable medium 1322 is illustrated as a single medium, the term "machine readable medium" may include a single medium or multiple media (e.g., a centralized or distributed database, and/or associated caches and servers) configured to store the one or more instructions 1324. The term "machine readable medium" may include any medium that is capable of storing, encoding, or carrying instructions for execution by the machine 1300 and that cause the machine 1300 to perform any one or more of the techniques of the present disclosure, or that is capable of storing, encoding, or carrying data structures used by or associated with such instructions. Non-limiting machine readable medium examples may include solid-state memories, and optical and magnetic media. In an example, a massed machine readable medium comprises a machine readable medium with a plurality of particles having invariant (e.g., rest) mass. Accordingly, massed machine-readable media are not transitory propagating signals. Specific examples of massed machine readable media may include: non-volatile memory, such as semiconductor memory devices (e.g., Electrically Programmable Read-Only Memory (EPROM), Electrically Erasable Programmable Read-Only Memory (EEPROM)) and flash memory devices; magnetic disks, such as internal hard disks and removable disks; magneto-optical disks; and CD-ROM and DVD-ROM disks.

Unless specifically stated otherwise, it is appreciated that throughout the description, discussions utilizing terms such as " receiving", "determining", "comparing ", "enabling", "maintaining," "identifying,", "obtaining", "accessing" or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

Additionally aspects and features of the present disclosure are set forth in the following numbered examples:
Example 1 is a method for presenting visual results of a particle beam treatment delivery, the method comprising: obtaining planning information of a planned particle beam treatment to treatment spots, the treatment spots located in a treatment area of a patient; obtaining delivery information of a delivered particle beam treatment to the treatment spots; determining an applicable dose deviation between a planned amount of radiation for the planned particle beam treatment and a delivered amount of radiation from the delivered particle beam treatment for each of the treatment spots; and presenting a visualization that represents the treatment spots and the applicable dose deviation between the planned amount of radiation and the delivered amount of radiation, for each of the treatment spots.
In Example 2, the subject matter of Example 1 optionally includes wherein the method further comprises: determining an applicable dose location deviation between a planned location of the planned particle beam treatment and a delivered location of the delivered particle beam treatment, for each of the treatment spots; wherein the visualization additionally represents the applicable dose location deviation between the planned location and the delivered location, using a directional indicator for respective spots of the treatment spots.
In Example 3, the subject matter of Example 2 optionally includes wherein if the applicable dose deviation exceeds a predetermined threshold for a respective spot, the directional indicator is presented adjacent to the respective spot, and the directional indicator points in a direction from the respective spot towards an area of planned delivery.
In Example 4, the subject matter of any one or more of Examples 1-3 optionally include wherein the visualization uses different colors among the treatment spots to represent an amount of the applicable dose deviation between the planned amount of radiation and the delivered amount of radiation.
In Example 5, the subject matter of Example 4 optionally includes wherein a first color is used to represent respective spots of the treatment spots that have no dose deviation within a predetermined range, wherein a second color is used to represent respective spots of the treatment spots that have a dose deviation outside the predetermined range with less delivered radiation than planned radiation, and wherein a third color is used to represent respective spots of the treatment spots that have a dose deviation outside the predetermined range with more delivered radiation than planned radiation.
In Example 6, the subject matter of any one or more of Examples 1-5 optionally include wherein the method further comprises: outputting the visualization in a graphical user interface, wherein the graphical user interface is configured to receive user interaction to change views and perspectives of the visualization.
In Example 7, the subject matter of Example 6 optionally includes wherein the visualization provides a three-dimensional representation that arranges respective spheres to represent each of the treatment spots.
In Example 8, the subject matter of Example 7 optionally includes wherein the graphical user interface is configured to receive additional user interaction to change visibility of the respective spheres, based on at least one criterion.
In Example 9, the subject matter of any one or more of Examples 1-8 optionally include wherein the planning information is provided from a treatment planning system, and wherein the treatment spots correspond to discrete areas of the planned particle beam treatment in a treatment space mapped by the treatment planning system.
In Example 10, the subject matter of any one or more of Examples 1-9 optionally include wherein the delivery information is provided from an oncology information system, and wherein the delivery information includes measurements for the delivered amount of radiation provided to the treatment spots.
Example 11 is a non-transitory computer-readable medium with instructions stored thereon that, when executed by a processor of a computing device, cause the processor to perform the methods of any of Examples 1 to 10.
Example 12 is a system for presenting visual results of a particle beam treatment delivery, the system comprising: processing circuitry; and memory, including instructions stored thereon, which, when executed by the processing circuitry, cause the processing circuitry to perform any of the methods of Examples 1 to 10.

According to another example, there is provided a computer program comprising instructions which, when the program is executed by a computer, configure or cause the computer to carry out any of the above examples. A computer program and/or the code for performing such methods may be provided to a system on one or more computer readable media or, more generally, a computer program product.

The examples described herein are computer-implemented methods. Since some methods in accordance with examples can be implemented by software, some examples encompass computer code provided to a general purpose computer on any suitable carrier medium. The carrier medium can comprise any storage medium such as a magnetic disk or device, optical disk, a programmable memory device, or any transient medium such as any signal, e.g., an electrical, optical or microwave signal. The carrier medium may comprise a non-transitory computer readable storage medium.

The present disclosure also relates to a system for performing the operations herein. This system may be specially constructed for the required purposes, or it may comprise a general purpose computer selectively activated or reconfigured by a computer program stored in the computer. The order of execution or performance of the operations in embodiments of the disclosure illustrated and described herein is not essential, unless otherwise specified. That is, the operations may be performed in any order, unless otherwise specified, and embodiments of the disclosure may include additional or fewer operations than those disclosed herein. For example, it is contemplated that executing or performing a particular operation before, contemporaneously with, or after another operation is within the scope of aspects of the disclosure.

In view of the above, it will be seen that the several objects of the disclosure are achieved and other advantageous results attained. Having described aspects of the disclosure in detail, it will be apparent that modifications and variations are possible without departing from the scope of aspects of the disclosure as defined in the appended claims. As various changes could be made in the above constructions, products, and methods without departing from the scope of aspects of the disclosure, it is intended that all matter contained in the above description and shown in the accompanying drawings shall be interpreted as illustrative and not in a limiting sense.

The above description is intended to be illustrative, and not restrictive. For example, the above-described examples (or one or more aspects thereof) may be used in combination with each other. In addition, many modifications may be made to adapt a particular situation or material to the teachings of the disclosure without departing from its scope. While the dimensions, types of materials and coatings described herein are intended to define the parameters of the disclosure, they are by no means limiting and are exemplary embodiments. Many other embodiments will be apparent to those of skill in the art upon reviewing the above description. The scope of the disclosure should, therefore, be determined with reference to the appended claims, along with the full scope of equivalents to which such claims are entitled.

Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description, with each claim standing on its own as a separate embodiment. The scope of the disclosure should be determined with reference to the appended claims, along with the full scope of equivalents to which such claims are entitled.

## Claims

1. A method for presenting visual results of a particle beam treatment delivery, the method comprising:
obtaining planning information of a planned particle beam treatment to treatment spots, the treatment spots located in a treatment area of a patient;
obtaining delivery information of a delivered particle beam treatment to the treatment spots;
determining an applicable dose deviation between a planned amount of radiation for the planned particle beam treatment and a delivered amount of radiation from the delivered particle beam treatment for each of the treatment spots; and
presenting a visualization that represents the treatment spots and the applicable dose deviation between the planned amount of radiation and the delivered amount of radiation, for each of the treatment spots.

2. The method of claim 1, wherein the method further comprises:
determining an applicable dose location deviation between a planned location of the planned particle beam treatment and a delivered location of the delivered particle beam treatment, for each of the treatment spots;
wherein the visualization additionally represents the applicable dose location deviation between the planned location and the delivered location, using a directional indicator for respective spots of the treatment spots.

3. The method of claim 2, wherein if the applicable dose deviation exceeds a predetermined threshold for a respective spot, the directional indicator is presented adjacent to the respective spot, and the directional indicator points in a direction from the respective spot towards an area of planned delivery.

4. The method of any of claims 1 to 3, wherein the visualization uses different colors among the treatment spots to represent an amount of the applicable dose deviation between the planned amount of radiation and the delivered amount of radiation.

5. The method of claim 4,
wherein a first color is used to represent respective spots of the treatment spots that have no dose deviation within a predetermined range,
wherein a second color is used to represent respective spots of the treatment spots that have a dose deviation outside the predetermined range with less delivered radiation than planned radiation, and
wherein a third color is used to represent respective spots of the treatment spots that have a dose deviation outside the predetermined range with more delivered radiation than planned radiation.

6. The method of any of claims 1 to 5, wherein the method further comprises:
outputting the visualization in a graphical user interface,
wherein the graphical user interface is configured to receive user interaction to change views and perspectives of the visualization.

7. The method of claim 6, wherein the visualization provides a three-dimensional representation that arranges respective spheres to represent each of the treatment spots.

8. The method of claim 7, wherein the graphical user interface is configured to receive additional user interaction to change visibility of the respective spheres, based on at least one criterion.

9. The method of any of claims 1 to 8, wherein the planning information is provided from a treatment planning system, and wherein the treatment spots correspond to discrete areas of the planned particle beam treatment in a treatment space mapped by the treatment planning system.

10. The method of any of claims 1 to 9, wherein the delivery information is provided from an oncology information system, and wherein the delivery information includes measurements for the delivered amount of radiation provided to the treatment spots.

11. A non-transitory computer-readable medium with instructions stored thereon that, when executed by a processor of a computing device, cause the processor to perform the method of any of claims 1 to 10.

12. A computing system configured for presenting visual results of a particle beam treatment delivery, the system comprising:
at least one processor; and
memory, including instructions stored thereon, which, when executed by the at least one processor, cause the computing system to perform the method of any of claims 1 to 10.
